# EUROPÄISCHE PATENTSCHRIFT

(11) **EP 3 541 445 B1**
(45) Veröffentlichungstag und Bekanntmachung des Hinweises auf die Patenterteilung: **02.09.2020**
(21) Anmeldenummer: 17800531.0
(22) Anmeldetag: 17.11.2017
(51) Int. Cl.: A61M 1/16

(54) **DIALYSEMASCHINE UND REINIGUNGSVERFAHREN FÜR DIE DIALYSEMASCHINE**
DIALYSIS MACHINE, AND CLEANING PROCESS FOR SAID DIALYSIS MACHINE
MACHINE DE DIALYSE ET PROCÉDÉ DE NETTOYAGE POUR CETTE MACHINE DE DIALYSE

(30) Priorität: 19.11.2016 DE 102016013875
(43) Veröffentlichungstag der Anmeldung: 25.09.2019
(73) Patentinhaber: Fresenius Medical Care Deutschland GmbH, 61352 Bad Homburg (DE)
(72) Erfinder: DANIEL, Pia, 78351 Bodman (DE); GOESSMANN, Stephan, 61381 Friedrichsdorf (DE); KARL, Klaus, 63755 Alzenau (DE); VERCH, Georg, 65191 Wiesbaden (DE)
(74) Vertreter: Schön, Andrea
(86) Internationale Anmeldenummer: PCT/EP2017/079566
(87) Internationale Veröffentlichungsnummer: WO 2018/091642

(56) Entgegenhaltungen:
- EP-A1- 0 622 086
- WO-A1-2007/148442
- WO-A1-2012/166377
- DE-A1- 10 256 584
- DE-A1-102011 008 223

## Beschreibung

### Technisches Gebiet

Die Erfindung betrifft eine Dialysemaschine mit geringerem Wartungsbedarf und ein Reinigungsverfahren zur Verringerung des Wartungsbedarfes.

### Hintergrund

In der chronischen Hämodialyse zur Behandlung von Niereninsuffizienz werden pro Behandlung circa 120-180I Dialyselösung benötigt.

Diese Dialyselösung wird üblicherweise vor Ort von der Behandlungsmaschine durch Verdünnung und Mischung eines sauren und eines basischen Konzentrates mit Dialysewasser (Permeat) hergestellt.

Das basische Konzentrat besteht aus reinem Bicarbonat und wird fast ausschließlich als Trockenkonzentrat bereitgestellt.

Das saure Konzentrat ist zumeist eine Mischung aus Kochsalz (circa 75 Gew.%), einer Säure, KCl, CaCl2, MgCl2 und Glucose. Dieses Konzentrat wird üblicherweise als Flüssigkonzentrat in Gebinden, wie z. B. in Kanistern oder Beutel, geliefert, die eine ausreichende Konzentratmenge für 1-2 Behandlungen, also 5-10 Liter Konzentrat, liefern. Diese Kanister werden von dem Pflegepersonal direkt zur Behandlungsmaschine gebracht. Die Behandlungsmaschine weist dann z.B. einen Konzentrat-Ansaugstab auf, mit dem das Konzentrat aus dem Kanister entnommen wird.

Dialysekliniken, in denen viele Patienten gleichzeitig behandelt werden, besitzen häufig eine zentrale Konzentratversorgung. Diese zentralen Konzentratversorgungen liefern das Flüssigkonzentrat z.B. aus einer größeren Verpackungseinheit (z.B. 300-400I) über eine oder mehrere Ringleitungen an die einzelnen Behandlungsplätze. Dort sind die Behandlungsmaschinen dann über Verbindungsschläuche mit dieser Ringleitung verbunden. Jede Behandlungsmaschine kann über getrennte Anschlüsse gleichzeitig mit zwei verschiedenen Konzentrat-Ringleitungen verbunden sein.

Diese zentralen Konzentratversorgungen bieten den Vorteil, dass zum einen die schweren Kanister nicht zum Behandlungsplatz gebracht werden müssen und zum anderen durch die größeren Gebinde das Abfallaufkommen vermindert wird.

Der Vorteil der Verwendung von einzelnen Kanistern ist die Möglichkeit der Anpassung der Zusammensetzung der Dialyselösung an die individuellen Anforderungen des einzelnen Patienten, der gerade behandelt wird. So können abhängig vom Patienten unterschiedliche Konzentrationen der Elektrolyte, unterschiedliche Säuren oder auch unterschiedliche Konzentrationen an Glucose indiziert sein.

Im Unterschied dazu kann pro vorhandener Ringleitung nur ein einheitliches Konzentrat bereitgestellt werden, das nicht unbedingt für alle Patienten optimiert ist. Die Anzahl der vorhandenen Ringleitungen ist üblicherweise auf 1-2 begrenzt. Wenn nun zur Erzeugung der Dialyselösung häufig Konzentrat-Kanister verwendet werden und/oder mehrere Konzentrat-Ringleitungen mit der Dialysemaschine verbunden sind, steht der Inhalt der dann jeweils ungenutzten Konzentrat-Verbindungsschläuche über einen potenziell längeren Zeitraum, was zu folgendem Problem führen kann.

Der Verbindungschlauch von der Behandlungsmaschine zur Ringleitung der zentralen Konzentratversorgung besteht aus Kunststoff. Durch diese Kunststoffschläuche kann in geringem Maße Wasser diffundieren, wodurch es, insbesondere bei einer längeren Nutzungspause, zu einer Aufkonzentration des in dem Schlauch vorhandenen Konzentrates kommt. Da die Flüssigkonzentrate als nahezu gesättigte Salzlösungen vorliegen, kann es deshalb zur Bildung von Salzkristallen in dem Schlauch kommen. Diese Salzkristalle werden dann bei der nächsten Verwendung der zentraten Konzentratversorgung in die Maschine gespült und können dort zu Störungen führen, die einen außerplanmäßigen Service der Maschine notwendig machen.

In der EP0622086 wird eine Dialysemaschine beschrieben, die zur Sicherstellung der Abtrennung der Hydraulik der Dialysemaschine von der Zentralversorgungseineheit während der Desinfektion der Dialysemaschine, in der Konzentratleitung der Dialysemaschine zwei Ventile aufweist. Zwischen diesen wird ein drucküberwachtes Puffervolumen bereitgestellt. Sinkt der Druck ab, kann auf eine Leakage rückgeschlossen werden.

Die Aufgabe der vorliegenden Erfindung besteht in der Bereitstellung einer Behandlungsmaschine für die Hämodialyse, bei der die Kristallbildung in den Verbindungsschläuchen der Behandlungsmaschine zur zentralen Konzentratversorgung vermieden wird.

### Zusammenfassung der Erfindung

Nach der Lehre der vorliegenden Erfindung wird diese Aufgabe gelöst durch eine Vorrichtung nach Ansprüchen 1 bis 8 sowie durch ein Verfahren nach den Ansprüchen 9 bis 13.

In Einklang mit der vorliegenden Lehre wird eine Dialysebehandlungseinheit mit eine Zubereitungseinheit für Dialyselösung offenbart. Diese Zubereitungseinheit dient zur Herstellung einer gebrauchsfertigen Dialyselösung aus zumindest einem Konzentrat und reinem Dialysewasser (Permeat). Das Konzentrat kann über eine zentrale Konzentratversorgung bereitgestellt werden. Diese zentrale Konzentratversorgung stellt Flüssigkonzentrat über ein Leitungsmittel, z.B. eine Ringleitung, an mehreren Behandlungsplätzen mit Dialysebehandlungseinheiten bereit. Die Dialysebehandlungseinheit weist ein Anschlussmittel auf, das an das Leitungsmittel der zentralen Konzentratversorgung anschließbar ist. Dieses Anschlussmittel ist auf der Seite der Dialysebehandlungseinheit mit einer Konzentratleitung der Zubereitungseinheit verbunden. In dieser Konzentratleitung ist zumindest ein Ventil vorgesehen, mittels dessen die Überführung von Konzentrat aus dem Anschlussmittel in die Konzentratleitung der Zubereitungseinheit durch eine Steuerungseinheit steuerbar ist. Diese Steuerungseinheit ist dabei dazu konfiguriert, während einem Spül- und/oder Reinigungsverfahren die Überführung von Flüssigkonzentrat aus dem Anschlussmittel in die Konzentratleitung zu veranlassen.

In dem Anschlussmittel wird das Konzentrat, das aus dem Anschlussmittel in die Dialysemaschine geflossen ist, durch frisches Konzentrat aus dem Leitungsmittel der zentralen Konzentratversorgung ersetzt. Da diese Spül- und/oder Reinigungsverfahren regelmäßig durchgeführt werden, wird das Flüssigkonzentrat im Anschlussmittel unabhängig von der verwendeten Konzentratquelle regelmäßig ersetzt. Die Aufkonzentrierung des Konzentrates und die Bildung von Kristallen im Anschlussmittel werden damit vermieden.

Die Überführung des Konzentrates aus dem Anschlussmittel in die Konzentratleitung kann dabei durch das Öffnen des zumindest einen Ventils in der Konzentratleitung erzeugt werden, wenn in dem Anschlussmittel ein höherer Druck besteht als in der Konzentratleitung zwischen dem zumindest einen Ventil und der Zubereitungseinheit. Das Leitungsmittel der zentralen Konzentratversorgung steht dabei standardmäßig unter einem Überdruck. Dieser Überdruck kann z.B. 0,05 bis 2 bar betragen. In der Konzentratleitung zwischen dem zumindest einen Ventil und der Zubereitungseinheit kann z.B. ein Unterdruck von -0,1 bar oder weniger bestehen.

In der Konzentratleitung können auch mehrere Ventile in Reihe geschaltet vorgesehen sein, die dann gleichzeitig geöffnet werden können. Bei Öffnung der Ventile erfolgt ein Druckausgleich durch Überführung des Konzentrates aus dem Anschlussmittel in die Konzentratleitung.

Die Öffnung des Ventiles oder der Ventile kann dabei über einen Zeitraum von 0,5 bis 5 s erfolgen, so dass eine genügende Konzentratmenge transportiert wird, z.B. 12ml.

Die überführte Konzentratmenge hängt z.B. von der Länge des Anschlussmittels, von dem Druckunterschied, der Viskosität des Konzentrates und der Nachgiebigkeit der zentralen Konzentratversorgung ab. Bei einem Druck in der Konzentratleitung des Dialysegerätes von - 0,2bar und einem 3 m langen Anschlussmittel werden zirka 12 Milliliter Konzentrat pro Sekunde angesaugt. Die Ventilöffnung kann also z.B. 1s betragen.

Die Ventilöffnung und damit die Überführung des Konzentrates kann dabei vorzugsweise in einer frühen Phase des Spül- oder Reinigungsverfahren erfolgen, z.B. Spül- oder Reinigungslösungen in die Konzentratleitung gefördert werden. Dadurch wird gewährleistet, dass Kristalle, die z.B. zusammen mit dem Konzentrat in die Konzentratleitung gelangen, zuverlässig ausgespült werden. Außerdem werden für den Fall, dass nach Spüllösungen, z.B. Dialysewasser (Permeat), Reinigungslösungen oder Desinfektionslösungen in die Konzentratleitung gefördert werden, chemische Reaktionen zwischen der Desinfektionslösung und dem Konzentrat vermieden.

Das Anschlussmittel kann durch einen Kunststoffschlauch gebildet werden, z.B. einen PVC-Schlauch.

Bei dem Spül- oder Reinigungsverfahren kann es sich um ein Desinfektionsverfahren handeln.

Weiterhin betrifft die Erfindung ein Spülverfahren für eine Dialysebehandlungseinheit. Dieses Spülverfahren dient als Vorbereitungsverfahren der Dialysebehandlungseinheit, das regelmäßig zwischen zwei Behandlungstagen durchgeführt wird um für die nächste Behandlung eine saubere, desinfizierte Dialysebehandlungseinheit bereitzustellen. Zusätzlich zu den Spülschritten mit Dialysewasser (Permeat) oder Reinigungslösungen oder Desinfektionslösungen umfasst das Spülverfahren auch einen Spülschritt für das Anschlussmittel an die zentrale Konzentratversorgung. Bei dem Spülverfahren für die Dialysebehandlungseinheit, umfassend eine Zubereitungseinheit für Dialyselösung mit einer Konzentratleitung aufweisend zumindest ein Ventil und mit wenigstens einem Anschlussmittel zum Anschließen der Konzentratleitung an ein Leitungsmittel einer zentralen Konzentratversorgungseinheit, wird zunächst Konzentrat aus dem Anschlussmittel in die Konzentratleitung transportiert, um eine Kristallbildung in dem Anschlussmittel zu verhindern, und danach erfolgen die Spülschritte zur Reinigung oder zur Reinigung und Desinfektion der Dialysebehandlungseinheit.

Der Überführung des Konzentrates aus dem Anschlussmittel in die Konzentratleitung kann durch Bereitstellung eines Ventiles in der Konzentratleitung, Einstellung eines Unterdrucks in der Konzentratleitung zwischen diesem Ventil und der Aufbereitungseinheit und die Öffnung dieses Ventiles für einen vorbestimmten Zeitraum erfolgen. Im Zuge des Druckausgleiches wird dann ein bestimmtes Volumensegment Flüssigkonzentrat aus dem Anschlussmittel in die Konzentratleitung überführt. Nach dem Schließen des Ventiles wird das aus dem Anschlussmittel geflossene Flüssigkonzentrat durch frisches Konzentrat aus der zentralen Konzentratversorgungseinheit ersetzt, das die korrekte Zusammensetzung aufweist. Die Aufkonzentrierung des Konzentrates im Anschlussmittel zur zentralen Konzentratversorgung wird vermieden. Der in der Konzentratleitung eingestellte Unterdruck kann -0,1 bar oder weniger betragen. Die Ventilöffnung kann für 0,5 bis 5s erfolgen. Bei dem Spülverfahren kann es sich um ein Verfahren zur Desinfektion der Dialysebehandlungseinheit handeln.

### Kurzbeschreibung der Zeichnungen

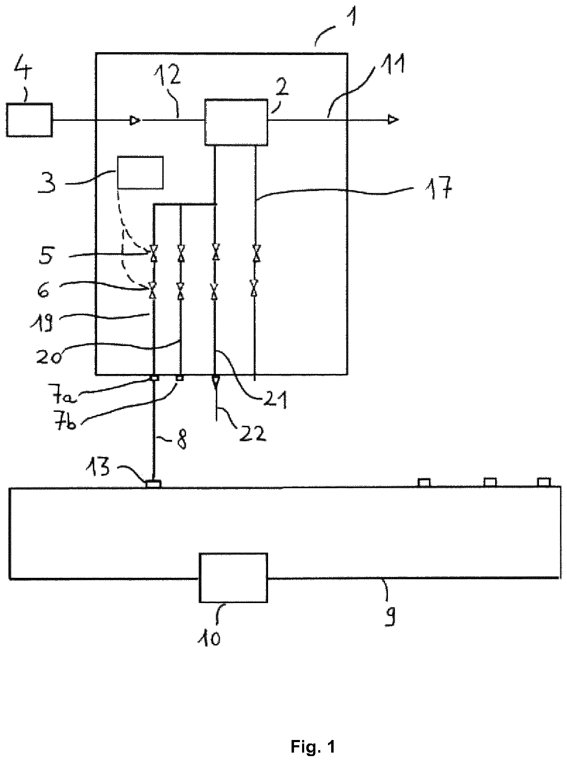

Figur 1 zeigt schematisch den Aufbau einer Dialysebehandlungseinheit.

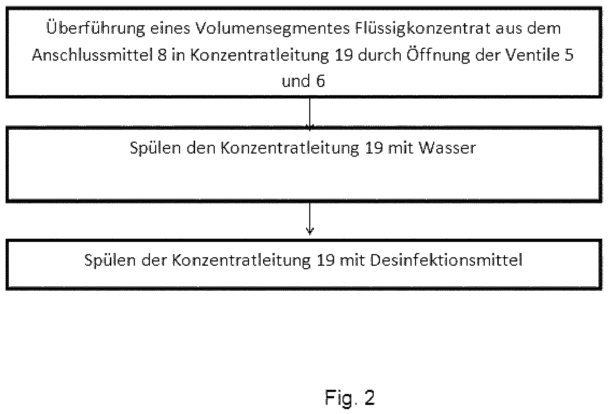

Figur 2 zeigt schematisch den Verfahrensablauf.

### Detaillierte Beschreibung eines Ausführungsbeispiels

Die Dialysebehandlungseinheit 1 stellt mittels einer Zubereitungseinheit 2 Dialyselösung online aus Dialysewasser (Permeat) und Konzentraten her. Das Dialysewasser (Permeat) wird dabei über die Leitung 12 von einer Reverse-Osmose-Anlage 4 zu der Zubereitungseinheit 2 geliefert. Die Dialyselösung wird üblicherweise durch Verdünnung und Mischung eines sauren und eines basischen Konzentrates mit Dialysewasser (Permeat) hergestellt. Dabei ist für das basische Konzentrat eine Konzentratleitung 17 vorgesehen. Es ist allerdings auch möglich, dass für das basische Konzentrat eine Verbindung zu einer zentralen Konzentratversorgung vorgesehen ist (nicht gezeigt). Die gebrauchsfertige Dialyselösung wird dann zu einem Behandlungsmodul geliefert, von wo die Dialyselösung dann über die Leitung 11 z.B. zu einem Dialysefilter (nicht gezeigt) weitergeleitet wird.

Für das saure Konzentrat sind bei modernen Dialysebehandlungseinheiten meistens mehrere Optionen wählbar. So kann das saure Konzentrat mit einem Konzentratansaugstab 22 aus einem Kanister entnommen werden und über eine Konzentratleitung 21 zu der Zubereitungseinheit 2 transportiert werden. Das saure Konzentrat kann aber auch von einer zentralen Konzentratversorgung 10 zur Verfügung gestellt werden. Die Dialysebehandlungseinheit 1 weist Konnektoren 7a und 7b für die Verbindung mit einer zentralen Konzentratversorgung 10 auf. An den Konnektor 7a ist ein Ende eines Anschlussmittels 8, z.B. ein Kunststoffschlauches, konnektiert. Das zweite Ende des Anschlussmittels 8 ist mit einem Konnektor 13 der Ringleitung 9 der zentralen Konzentratversorgung 10 verbunden. Während in der Ringleitung 9 stets ein Flüssigkeitsaustausch stattfindet, ist der Durchgang im Anschlussmittel 8 nur dann gegeben, wenn Konzentrat aus der zentralen Konzentratversorgung 10 bezogen wird. Die Dialysebehandlungseinheit 1 weist eine Steuerungseinheit 3 auf, die dazu konfiguriert ist, während eines Reinigungsverfahrens die Ventile 5 und 6 zu öffnen. Da in der Ringleitung 9 einer zentralen Konzentratversorgungseinheit 10 standardmäßig ein Überdruck von 0,5 bis 2,0 bar herrscht und in der Konzentratleitung 19 hinter den Ventilen 5 und 6 ein Unterdruck von -0,2 bar, wird mit der Öffnung der Ventile 5 und 6 ein bestimmtes Flüssigkeitsvolumen in die Konzentratleitung 19 gefördert. So werden bei einer Ventilöffnung für eine Sekunde circa 12 ml Konzentrat gefördert. Da die Reinigungsverfahren regelmäßig, d.h. immer wenn die Dialysebehandlungseinheit 1 verwendet wird, durchgeführt werden, findet ein regelmäßiger Austausch des Konzentrates im Anschlussmittel 8 statt. Die Bildung von Niederschlägen in dem Anschlussmittel 8 wird vermieden. Weitere in dem Spülverfahren verwendete Lösungen, z.B. Desinfektionsmittel, können auch zu der Zubereitungseinheit 2 zugeleitet und von dort verteilt werden (nicht gezeigt).

Das Spülverfahren weist im Unterschied zu den bekannten Spülverfahren einen zusätzlichen Verfahrensschritt auf, nämlich der Überführung eines Volumensegmentes Flüssigkonzentrat aus dem Anschlussmittel 8 in die Konzentratleitung 19. Das Anschlussmittel 8 wird dann über die Ringleitung 9 der zentralen Konzentratversorgung mit frischem Flüssigkonzentrat versorgt. In der Dialysebehandlungseinheit werden anschließend die flüssigkeitsführenden Leitungen der Dialysebehandlungseinheit mit Dialysewasser (Permeat) gespült, um sie von dem Konzentrat freizuspülen. Daran anschließend werden die Leitungen dann mit Reinigungslösung oder Desinfektionslösung gespült und die weiteren Schritte zur Reinigung der Maschine durchgeführt. Durch den Zwischenspülschritt werden z.B. chemische Reaktionen des Flüssigkonzentrates mit Reinigungs- oder Desinfektionslösung vermieden.

## Patentansprüche

1. Dialysebehandlungseinheit (1) mit einer Zubereitungseinheit (2) für Dialyselösung mit wenigstens einem Anschlussmittel (8), mittels dessen eine Konzentratleitung (19) der Zubereitungseinheit (2) an ein Leitungsmittel (9) eines zentralen Konzentratversorgungssystems (10) anschließbar ist, mit wenigstens einem Ventil (5,6) in der Konzentratleitung (19), mittels dessen die Überführung von Konzentrat aus dem Anschlussmittel (8) in die Konzentratleitung (19) durch die Öffnung des Ventiles (5, 6) durch eine Steuerungseinheit (3) der Dialysebehandlunseinheit (1) steuerbar ist, **dadurch gekennzeichnet, dass** die Steuerungseinheit (3) dazu konfiguriert ist, während einem Spül- oder Reinigungsverfahren die Überführung von Konzentrat aus dem Anschlussmittel (8) in die Konzentratleitung (19) zu veranlassen um die Kristallbindung in dem Anschlussmittel (8) zu verhindern.

2. Dialysebehandlungseinheit (1) nach Anspruch 1, wobei in der Konzentratleitung (19) zwischen dem Ventil (5,6) und der Zubereitungseinheit (2) ein Unterdruck besteht und die Überführung des Konzentrates alleine durch die Öffnung des Ventils (5, 6) erfolgt.

3. Dialysebehandlungseinheit (1) nach Anspruch 2, wobei der Unterdruck in der Konzentratleitung (19) zwischen dem Ventil (5, 6) und der Zubereitungseinheit (2) -0,1 bar oder weniger und beträgt.

4. Dialysebehandlungseinheit (1) nach Anspruch 2 oder 3, wobei im Anschlussmittel (8) ein Überdruck von 0,05 bis 2 bar besteht.

5. Dialysebehandlungseinheit (1) nach Anspruch 2, wobei die Steuerungseinheit (2) das Ventil (5, 6) für einen Zeitraum von 0,5-5s öffnet.

6. Dialysebehandlungseinheit (1) nach einem der vorangehenden Ansprüche, wobei die Steuerungseinheit (3) das Ventil (5, 6) öffnet, bevor im Spül- oder Reinigungsverfahren Spül- oder Reinigungslösungen in die Konzentratleitung (19)gefördert werden.

7. Dialysebehandlungseinheit (1) nach einem der vorangehenden Ansprüche, wobei das Anschlussmittel (8) einen Kunststoffschlauch umfasst.

8. Dialysebehandlungseinheit (1) nach einem der vorangehenden Ansprüche, wobei das Spül- oder Reinigungsverfahren ein Desinfektionsverfahren ist.

9. Spülverfahren für eine Dialysebehandlungseinheit (1) umfassend eine Zubereitungseinheit (2) für Dialyselösung mit einer Konzentratleitung (19) aufweisend ein Ventil (5, 6) und mit wenigstens einem Anschlussmittel (8) zum Anschließen der Konzentratleitung (19) an ein Leitungsmittel (9) einer zentralen Konzentratversorgungseinheit (10), **dadurch gekennzeichnet, dass** vor Spülung der Konzentratleitung (19) mit Spül- oder Reinigungslösung eine Steuerungseinheit (3) die Öffnung des Ventils (5, 6) in der Konzentratleitung (19) veranlasst, wodurch Flüssigkonzentrat aus dem Anschlussmittel (8) in die Dialysebehandlungseinheit (1) überführt wird, um eine Kristallbildung in dem Anschlussmittel (8) zu verhindern, und danach Spülschritte zur Reinigung oder zur Reinigung und Desinfektion der Dialysebehandlungseinheit erfolgen.

10. Spülverfahren nach Anspruch 9, wobei vor Spülung mit Reinigungs- oder Desinfektionsmittel eine Spülung mit Dialysewasser (Permeat) erfolgt.

11. Spülverfahren nach Anspruch 9 oder 10, wobei in der Konzentratleitung (19) zwischen dem Ventil (5, 6) und der Aufbereitungseinheit (2) ein Unterdruck eingestellt ist.

12. Spülverfahren nach Anspruch 11, wobei in der Konzentratleitung (19) ein Unterdruck von -0,1 bar oder weniger eingestellt wird.

13. Spülverfahren nach Anspruch 11 oder 12, wobei. das Ventil (5, 6) für 0,5 bis 5s geöffnet wird.

14. Spülverfahren nach einem der Ansprüche 9 bis 13, wobei das Spülverfahren zur Desinfektion der Dialysebehandlungseinheit (1) angewendet wird.

## Claims

1. Dialysis treatment unit (1) having a preparation unit (2) for dialysis solution, having at least one connecting means (8) by which a concentrate line (19) of the preparation unit (2) can be connected to a line means (9) of a central concentrate supply system (10), having at least one valve (5, 6) in the concentrate line (19), by means of which the transfer of concentrate from the connecting means (8) into the concentrate line (19) can be controlled by the opening of the valve (5, 6) by a control unit (3) of the dialysis treatment unit (1), **characterized in that** the control unit (3) is configured to cause the transfer of concentrate from the connecting means (8) into the concentrate line (19) during a rinsing or cleaning process in order to prevent crystal formation in the connecting means (8).

2. Dialysis treatment unit (1) according to Claim 1, wherein a negative pressure prevails in the concentrate line (19) between the valve (5, 6) and the preparation unit (2), and the transfer of the concentrate takes place solely by the opening of the valve (5, 6).

3. Dialysis treatment unit (1) according to Claim 2, wherein the negative pressure in the concentrate line (19) between the valve (5, 6) and the preparation unit (2) is -0.1 bar or less.

4. Dialysis treatment unit (1) according to Claim 2 or 3, wherein a positive pressure of 0.05 to 2 bar prevails in the connecting means (8).

5. Dialysis treatment unit (1) according to Claim 2, wherein the control unit (2) opens the valve (5, 6) for a period of 0.5-5 s.

6. Dialysis treatment unit (1) according to one of the preceding claims, wherein the control unit (3) opens the valve (5, 6) before rinsing or cleaning solutions are delivered into the concentrate line (19) in the rinsing or cleaning process.

7. Dialysis treatment unit (1) according to one of the preceding claims, wherein the connecting means (8) comprises a plastic tubing.

8. Dialysis treatment unit (1) according to one of the preceding claims, wherein the rinsing or cleaning process is a disinfection process.

9. Rinsing process for a dialysis treatment unit (1) comprising a preparation unit (2) for dialysis solution, having a concentrate line (19) with a valve (5, 6) and having at least one connecting means (8) for connecting the concentrate line (19) to a line means (9) of a central concentrate supply unit (10), **characterized in that**, before the concentrate line (19) is rinsed with rinsing or cleaning solution, a control unit (3) causes the valve (5, 6) in the concentrate line (19) to open, as a result of which liquid concentrate is transferred from the connecting means (8) into the dialysis treatment unit (1) in order to prevent crystal formation in the connecting means (8), and then rinsing steps are carried out to clean or to clean and disinfect the dialysis treatment unit.

10. Rinsing process according to Claim 9, wherein rinsing with dialysis water (permeate) takes place before rinsing with cleaning agent or disinfectant.

11. Rinsing process according to Claim 9 or 10, wherein a negative pressure is established in the concentrate line (19) between the valve (5, 6) and the preparation unit (2).

12. Rinsing process according to Claim 11, wherein a negative pressure of -0.1 bar or less is established in the concentrate line (19).

13. Rinsing process according to Claim 11 or 12, wherein the valve (5, 6) is opened for 0.5 to 5 s.

14. Rinsing process according to one of Claims 9 to 13, wherein the rinsing process is used for disinfecting the dialysis treatment unit (1).

## Revendications

1. Unité de traitement de dialyse (1) comprenant une unité de préparation (2) pour une solution de dialyse, comprenant au moins un moyen de raccordement (8) au moyen duquel une conduite de concentré (19) de l'unité de préparation (2) peut être raccordée à un moyen de conduite (9) d'un système central d'alimentation en concentré (10), comprenant au moins une soupape (5, 6) dans la conduite de concentré (19), au moyen de laquelle le transfert de concentré depuis le moyen de raccordement (8) jusque dans la conduite de concentré (19) à travers l'ouverture de la soupape (5, 6) peut être commandé par une unité de commande (3) de l'unité de traitement de dialyse (1),
**caractérisée en ce que** l'unité de commande (3) est configurée pour permettre le transfert de concentré depuis le moyen de raccordement (8) jusque dans la conduite de concentré (19) au cours d'un procédé de rinçage ou de nettoyage afin d'empêcher la formation de cristaux dans le moyen de raccordement (8).

2. Unité de traitement de dialyse (1) selon la revendication 1, dans laquelle une dépression existe dans la conduite de concentré (19) entre la soupape (5, 6) et l'unité de préparation (2) et le transfert du concentré s'effectue uniquement par l'ouverture de la soupape (5, 6).

3. Unité de traitement de dialyse (1) selon la revendication 2, dans laquelle la dépression dans la conduite de concentré (19) entre la soupape (5, 6) et l'unité de préparation (2) est de -0,1 bar ou moins.

4. Unité de traitement de dialyse (1) selon la revendication 2 ou 3, dans laquelle une surpression de 0,05 à 2 bars existe dans le moyen de raccordement (8).

5. Unité de traitement de dialyse (1) selon la revendication 2, dans laquelle l'unité de commande (2) ouvre la soupape (5, 6) pendant un intervalle de temps de 0,5 à 5 s.

6. Unité de traitement de dialyse (1) selon l'une quelconque des revendications précédentes, dans laquelle l'unité de commande (3) ouvre la soupape (5, 6) avant que des solutions de rinçage ou de nettoyage ne soient refoulées dans la conduite de concentré (19) au cours du procédé de rinçage ou de nettoyage.

7. Unité de traitement de dialyse (1) selon l'une quelconque des revendications précédentes, dans laquelle le moyen de raccordement (8) comprend un tuyau en plastique.

8. Unité de traitement de dialyse (1) selon l'une quelconque des revendications précédentes, dans laquelle le procédé de rinçage ou de nettoyage est un procédé de désinfection.

9. Procédé de rinçage pour une unité de traitement de dialyse (1) comprenant une unité de préparation (2) pour une solution de dialyse avec une conduite de concentré (19) présentant une soupape (5, 6) et avec au moins un moyen de raccordement (8) pour le raccordement de la conduite de concentré (19) à un moyen de conduite (9) d'une unité centrale d'alimentation en concentré (10), **caractérisé en ce qu'**avant le rinçage de la conduite de concentré (19) avec la solution de rinçage ou de nettoyage, une unité de commande (3) permet l'ouverture de la soupape (5, 6) dans la conduite de concentré (19), de sorte que le concentré fluide soit transféré depuis le moyen de raccordement (8) jusque dans l'unité de traitement de dialyse (1) afin d'empêcher une formation de cristaux dans le moyen de raccordement (8), puis des étapes de rinçage sont effectuées pour nettoyer ou pour nettoyer et désinfecter l'unité de traitement de dialyse.

10. Procédé de rinçage selon la revendication 9, dans lequel, avant le rinçage avec l'agent de nettoyage ou de désinfection, un rinçage est effectué avec de l'eau de dialyse (perméat).

11. Procédé de rinçage selon la revendication 9 ou 10, dans lequel une dépression est ajustée dans la conduite de concentré (19) entre la soupape (5, 6) et l'unité de préparation (2).

12. Procédé de rinçage selon la revendication 11, dans lequel une dépression de -0,1 bar ou moins est ajustée dans la conduite de concentré (19).

13. Procédé de rinçage selon la revendication 11 ou 12, dans lequel la soupape (5, 6) est ouverte pendant 0,5 à 5 s.

14. Procédé de rinçage selon l'une quelconque des revendications 9 à 13, le procédé de rinçage étant employé pour la désinfection de l'unité de traitement de dialyse (1).
